# EUROPEAN PATENT SPECIFICATION

(11) **EP 4 398 752 B1**
(45) Date of publication and mention of the grant of the patent: **12.11.2025**
(21) Application number: 22773309.4
(22) Date of filing: 02.09.2022
(51) Int. Cl.: A24F 40/42, A24F 40/48, A61M 15/06, A24F 40/85, A61M 11/04, A61M 15/00

(54) **AEROSOL-FORMING CARTRIDGE AND SYSTEM WITH NICOTINE GEL**
AEROSOLERZEUGUNGSPATRONE UND -SYSTEM MIT NIKOTINGEL
CARTOUCHE DE FORMATION D'AÉROSOL ET SYSTÈME AVEC GEL DE NICOTINE

(30) Priority: 09.09.2021 EP 21195767
(43) Date of publication of application: 17.07.2024
(73) Proprietor: Philip Morris Products S.A., 2000 Neuchâtel (CH)
(72) Inventor: FREDERICK, Guillaume, 2000 Neuchâtel (CH); MIRONOV, Oleg, 2000 Neuchâtel (CH); TAURINO, Irene, 2000 Neuchâtel (CH)
(74) Representative: Abitz & Partner
(86) International application number: PCT/IB2022/058268
(87) International publication number: WO 2023/037214

(56) References cited:
- WO-A1-2019/224531
- WO-A1-2020/176519
- WO-A1-2020/201706
- US-A- 4 183 365
- US-A1- 2020 288 780

## Description

The present disclosure relates to an aerosol-forming system with nicotine gel, the aerosol-forming system is configured to generate aerosol via heating of the nicotine gel.

Sensorial media of commercial electronic cigarettes are generally liquid (so called "e-liquids"). These e-liquids are typically contained in aerosol-forming articles or "pods" that are replaceable into the electronic cigarette. These pods include a heater in contact with a porous material or wick. This porous or wick transports e-liquid by capillarity action to the heater for vaporization into inhalation airflow of the electronic cigarettes.

A well-known problem of commercial electronic cigarettes is e-liquid leakage. Leakage occurs when there is too much e-liquid. Leakage occurs when the retention capability of the porous material does not prevent the e-liquid from flooding, especially in the case of full tank commercial electronic cigarettes. Leakage occurs when upon changes in environmental pressure during storage or transport. Leakage may also occur with temperature changes which affects the viscosity of the e-liquid and the retention capacity of the porous materials.

In addition, the heater of current commercial electronic cigarette systems is typically integrated in the replaceable cartridge or "pod" containing the e-liquid. Including the heater in the replaceable aerosol-forming article or "pod" increases the cost, complexity, and disposal costs of these replaceable aerosol-forming articles or "pods".

Document WO 2020/201706 A1 provides an aerosol provision system comprising: - an aerosol generating medium; - a source of energy for heating, wherein the source of energy for heating is configured to cause heating of the aerosol generating medium in a heating zone; - a movement mechanism arranged to move the aerosol generating medium; - an aerosol outlet through which an aerosol can flow to be inhaled by a user; and - a flow path arranged between the heating zone and the aerosol outlet such that aerosol formed from heated aerosol generating medium flows along the flow path, wherein the aerosol generating medium is translatable past the source of energy for heating, into the heating zone, such that respective portions of aerosol generating medium are individually presented to the source of energy for heating to form an aerosol; and wherein the aerosol generating medium is arranged to move along a line that is at an angle to the flow path of generated aerosol.

Document WO 2019/224531 A1 relates to a component for a vapour provision system comprising a reservoir for storing aerosolisable substrate material, the reservoir bounded by a boundary wall with at least one movable section configured to move to reduce a storage volume of the reservoir; an outlet in the reservoir for dispensing aerosolisable substrate material from the reservoir; a one-way outlet valve at the outlet configured to open for the dispensing of the aerosolisable substrate material; and a dispensing arrangement operable to increase pressure of aerosolisable substrate material in an outlet volume of the reservoir which is not bounded by the movable section, so as to open the outlet valve and dispense a portion of aerosolisable substrate material, the subsequent absence of the dispensed portion reducing the pressure to allow the movable section of the boundary wall to move to reduce the storage volume.

Document US 2020/288780 A1 relates to a vapor dispenser that includes a bifurcated housing having a body attached to a mouthpiece. A vaporizer cavity of the bifurcated body pneumatically communicates with an inlet orifice of the mouthpiece. A vaporizing component is received in the vaporizing cavity. An exhaust cavity of the bifurcated body pneumatically communicates with an outlet orifice of the mouthpiece. The exhaust cavity is pneumatically separated from the vaporizer cavity. A one-way valve is positioned in a selected one of: (i) the exhaust cavity; and (ii) outlet orifice and that: (i) opens in response to exhalation through the bifurcated mouthpiece; and (ii) closes in response to inhalation through the bifurcated mouthpiece. An air filter is received in the exhaust cavity.

Document WO 2020/176519 A1 relatest to a personal vaporization device for use with non-liquid vaporizable material, such as ground herbal material, is designed to provide for multiple vaporizing sessions with a single loading of the of the device. The device comprises an oven assembly including an oven for holding material for a single vaporizing session, and an electric heating element thermally coupled to the oven to heat the material in the oven. Vapors generated by the heated material travel through an air flow path for inhalation by a user. A hopper holds a supply of the material for at least one additional session, and the oven is resupplied for a new session by feeding material from the hopper via a flow path to the oven. The hopper can be designed to receive the vaporizable material directly or to receive a cartridge containing the supply of vaporizable material. A system for filling a plurality of cartridges is also provided.

Document US 4 183 365 A relates to a water pipe or bong for smoking tobacco comprising a base to which there is affixed one end of an upright, hollow cylinder, thereby leaving an open end which can be received against one's mouth so that smoke can be inhaled by applying a suction action to the interior of the cylinder. A revolver is axially aligned with the longitudinal centerline of the hollow cylinder with the cylinder being telescopingly received within the revolver and with the revolver being rotatably positioned about a medial portion of the cylinder. A plurality of radially spaced combustion chambers are formed within the revolver in which tobacco can be stored and combusted. The combustion chambers include an open end into which tobacco is charged and an outlet end through which smoke can flow. A passageway is formed between the outlet of the combustion chamber and the interior of the hollow cylinder so that air can be drawn into the combustion chamber, thereby burning the tobacco, so that smoke flows through the outlet into the interior of the chamber and into one's mouth.

There is a need for an aerosol-forming cartridge or system that is stable and does not leak liquid. There is a need for a replaceable aerosol-forming article that does not include a heating element. There is a need for an aerosol-forming cartridge or system that provides a metered dose of aerosol-forming substrate during operation.

It is desirable to provide an aerosol-forming system that utilizes a gel aerosol-forming substrate in the replaceable aerosol-forming article. It is desirable to provide an aerosol-forming system that includes the heating element in the aerosol-forming device that receives the replaceable aerosol-forming article. It is desirable to provide an aerosol-forming article that includes the rotating revolver element having two or more chambers that receive a metered dose of aerosol-forming substrate or gel in each chamber. It is desirable to provide an aerosol-forming article that includes the rotating revolver element having at least three chambers and includes a heating position, a cleaning position, and a deposition position.

This disclosure is directed to an aerosol-forming article or cartridge that includes an aerosol-forming substrate reservoir and a rotating revolver element having two or more chambers that receive a metered dose of aerosol-forming substrate or gel in each chamber. The rotating revolver element includes at least, a heating position and a deposition position, and optionally a cleaning position.

According to the present invention, there is provided an aerosol-forming cartridge as defined in the present claim 1.

According to another aspect of the present invention, an aerosol-forming system includes an aerosol-forming device and a replaceable cartridge or aerosol-forming article. The aerosol-forming device includes a device housing defining a device interior and a device exterior surface. The device exterior surface includes a cartridge receiving surface, and a heating element fixed to the cartridge receiving surface. The replaceable cartridge, described herein, is configured to mate with the cartridge receiving surface. The device heating element aligns with the revolver element in a gel heating position.

According to another aspect of the present invention, a method forming an aerosol includes inserting a replaceable cartridge, described herein onto the cartridge receiving surface of the aerosol-forming device, described herein. Then heating the aerosol-forming substrate contained within a chamber of the revolver element to form an aerosol in an inhalation airflow flowing through the cartridge. Then flowing the aerosol and inhalation airflow through a mouthpiece.

Advantageously, the aerosol-forming substrate in the aerosol-forming cartridge is a gel until the heating step and is thus stable and does not leak liquid during transport or storage or use of the aerosol-forming substate. In addition, the replaceable cartridge does not include a heating element and is less complex and less costly to produce. In addition, the replaceable cartridges provide a metered dose of aerosol-forming substrate per inhalation or puff as it is incremented forward from the deposition position to the heating position by the user. Advantageously, the revolver element has two or more chambers about the perimeter and the revolver element and rotates to provide, at least, a deposition position and a heating position to maintain the consistent operation of the aerosol-forming system, and optionally a cleaning position may be located between the heating position and the deposition position.

All values reported as a percentage are presumed to be weight percent based on the total weight.

All scientific and technical terms used herein have meanings commonly used in the art unless otherwise specified. The definitions provided herein are to facilitate understanding of certain terms used frequently herein.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all the listed elements or a combination of any two or more of the listed elements.

As used herein, "have", "having", "include", "including", "comprise", "comprising" or the like are used in their open-ended sense, and generally mean "including, but not limited to". It will be understood that "consisting essentially of", "consisting of", and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure, including the claims.

The term "substantially" as used here has the same meaning as "significantly," and can be understood to modify the relevant term by at least about 90 %, at least about 95 %, or at least about 98 %. The term "not substantially" as used here has the same meaning as "not significantly," and can be understood to have the inverse meaning of "substantially," i.e., modifying the relevant term by not more than 10%, not more than 5%, or not more than 2%.

The terms "upstream" and "downstream" refer to relative positions of elements of the inhaler device and inhaler systems described in relation to the direction of inhalation air flow as it is drawn through the body of the inhaler device and inhaler systems.

The term "solid" refers to a fundamental state of matter that does not expand to fill a space or flow, at a temperature of 25 degrees Celsius and one atmosphere pressure.

The term "nicotine" refers to nicotine and nicotine derivatives such as free-base nicotine, nicotine salts and the like.

As used herein, the terms "control electronics", "controller" and "processor" refer to any device or apparatus capable of providing computing capabilities and control capabilities suitable, or configurable to perform the methods, process, and techniques described herein such as, for example, microprocessors, digital signal processors (DSP), application specific integrated circuits (ASIC), field-programmable gate arrays (FPGA), equivalent discrete or integrated logic circuitry, or any combination thereof and of providing suitable data storage capabilities that includes any medium (for example, volatile or non-volatile memory, or magnetic recordable medium such as a disk or tape) containing digital bits (for example, encoded in binary or trinary) that may be readable and writeable.

The term "aerosol" is used here to refer to a suspension of solid particles or liquid droplets, or a combination of solid particles and liquid droplets in a gas. The gas may be air. The solid particles or liquid droplets may comprise one or more volatile flavor compounds. Aerosol may be visible or invisible. Aerosol may include vapors of substances that are ordinarily liquid or solid at room temperature. Aerosol may include vapors of substances that are ordinarily liquid or solid at room temperature, in combination with solid particles or in combination with liquid droplets or in combination with both solid particles and liquid droplets. The aerosol preferably comprises nicotine.

The term "aerosol-forming substrate" is used here to refer to a material capable of releasing one or more volatile compounds that can form an aerosol. In some embodiments, an aerosol-forming substrate may be heated to volatilize one or more components of the aerosol-forming substrate to form an aerosol. In some cases, volatile compounds may be released by a chemical reaction. Aerosol-forming substrate may be solid or liquid or may comprise both solid and liquid components such as a gel. Aerosol-forming substrate may be adsorbed, coated, impregnated, or otherwise loaded onto a carrier or support. Aerosol-forming substrate preferably comprises nicotine. Aerosol-forming substrate may comprise plant-based material. Aerosol-forming substrate may comprise tobacco. Aerosol-forming substrate may comprise a tobacco-containing material containing volatile tobacco flavor compounds, which are released from the aerosol-forming substrate upon heating. Aerosol-forming substrate may alternatively comprise a non-tobacco-containing material. Aerosol-forming substrate may comprise homogenized plant-based material. Aerosol-forming substrate may comprise homogenized tobacco material. Aerosol-forming substrate may comprise at least one aerosol-former. Aerosol-forming substrate may comprise other additives and ingredients, such as flavorants. The aerosol-forming substrate may comprise an active ingredient. The aerosol-forming substrate may be provided as part of an aerosol generating article. The aerosol-forming substrate may be provided in an aerosol-generating article.

The term "aerosol-generating article" is used here to refer to a disposable product capable of including (for example, holding, containing, having, or storing) aerosol-forming substrate. An aerosol-generating article may be capable of removably interfacing, or docking, with an aerosol generating device. This allows the aerosol-generating device to generate aerosol from the aerosol-forming substrate of the aerosol-generating article. An "aerosol-forming cartridge" is an example of an "aerosol-generating article".

The term "aerosol-generating device" is used here to refer to any device configured to be used or utilized with an aerosol forming substrate that releases volatile compounds to form an aerosol that may be inhaled by a user. The aerosol-generating device may be interfaced with an aerosol-generating article comprising the aerosol-forming substrate.

The term "heating element" is used here to refer to any device, apparatus, or portion thereof configured to provide heat, or heat energy, to an aerosol-generating article to release volatile compounds from the aerosol-generating article to form an aerosol.

The term "gel" refers to a gelled material. The gellified or gelled material may be a solid at room temperature. The gellified or gelled material may substantially maintain its shape and mass at room temperature. Room temperature in this context means 25 degrees Celsius. "Solid" in this context means that the material substantially maintains its shape and mass at room temperature and does not flow.

An aerosol-forming cartridge includes a housing defining a cartridge body and having a housing interior, an aerosol-forming substrate reservoir fixed within the housing interior, and a revolver element rotatably fixed to the housing interior and having two or more chambers disposed about the revolver element. The aerosol-forming substrate reservoir contains a volume of aerosol-forming substrate. The aerosol-forming substrate is a gel. A bias element applies force to the volume of aerosol-forming substrate. The aerosol-forming substrate reservoir having an outlet aperture. The two or more chambers of the revolver are sequentially aligned with the outlet aperture as the revolver element rotates.

The aerosol-generating substrate may include nicotine. Nicotine may be included in the aerosol-generating substrate in free base form or in salt form. The aerosol-generating substrate may comprise nicotine at a concentration of 1 wt-% or greater, 1.5 wt-% or greater, or 2 wt-% or greater. The aerosol-generating substrate may comprise nicotine at a 5 concentration of 4 wt-% or less, 3 wt-% or less, or 2.5 wt-% or less. In one embodiment, the aerosol-generating substrate comprises about 2 wt-% nicotine.

The aerosol-generating substrate is a gel that comprises an active ingredient and one or more gelling agents. The active ingredient may comprise nicotine. Nicotine may be included in the gel in a free base form or in salt form. The gel may comprise nicotine at a concentration of 1 wt-% or greater, 1.5 wt-% or greater, or 2 wt-% or greater. The gel may comprise nicotine at a concentration of 4 wt-% or less, 3 wt-% or less, or 2.5 wt-% or less. In one embodiment, the gel comprises about 2 wt-% nicotine. The one or more gelling agents may comprise a biopolymer. Examples of suitable biopolymers include polysaccharides, such as gellan gums (native, low acyl gellan gums and high acyl gellan gums), xanthan gum, alginates (alginic acid), agar (a mixture of agarose and agaropectin), agarose, guar gum, wax, and the like. The gel may comprise gelling agent at a concentration of 1 wt-% or greater, 1.5 wt-% or greater, or 2 wt-% or greater. The gel may comprise gelling agent at a concentration of 20 wt-% or less, 15 wt-% or less, or 10 wt-% or less.

The aerosol-generating substrate may include additional ingredients, such as flavorants, aerosol formers, water, compounds that assist gelling, and the like. The aerosol-generating substrate may comprise one or more flavorants. The one or more flavorants may comprise tobacco flavors. Examples of suitable tobacco flavors include synthetic and naturally derived tobacco components. Naturally derived tobacco components may include volatile flavors or flavor compounds obtained from tobacco plant material. Such components may be obtained by any suitable method, such as extraction, drying, milling, etc. Synthetic tobacco components may comprise flavor molecules found in tobacco leaves, such as beta-damascenone, alpha- and 3-oxo-alpha-ionone, beta- and 4-oxo-beta-ionone, theaspirone, 2-ethyl-3,5-dimethylpyrazine, phenylacetaldehyde, guaiacol, and furaneol. Other suitable flavorants include, for example, natural or synthetic menthol, peppermint, spearmint, coffee, tea, spices (such as cinnamon, clove, ginger, or combination thereof), cocoa, vanilla, fruit flavors, chocolate, eucalyptus, geranium, eugenol, agave, juniper, anethole, linalool, and any combination thereof.

The aerosol-generating substrate (for example, gel) may include glycerol. For example, the aerosol-generating substrate may comprise glycerol at a concentration of 50 wt-% or greater, 60 wt-% or greater, or 70 wt-% or greater. The aerosol-generating substrate may comprise glycerol at a concentration of 95 wt-% or less, 90 wt-% or less, or 80 wt-% or less. The aerosol-generating substrate may comprise glycerol at a concentration in a range from 50 wt-% to 95 wt-%, or in a range from 60 wt-% to 90 wt-%, or a range from 60 wt-% to 80 wt-%.

The aerosol-generating substrate may include water. For example, the aerosol-generating substrate may comprise water at a concentration of 10 wt-% or greater, 15 wt-% or greater, or 20 wt-% or greater. The aerosol-generating substrate may comprise water at a concentration of 25 wt-% or less, 20 wt-% or less, or 15 wt-% or less. In some embodiments, the aerosol-generating substrate is free or substantially free of water.

The aerosol-generating substrate may include a gel comprising one or more divalent cations. Examples of suitable divalent cations include compounds that comprise calcium, such as calcium lactate in solution. The divalent cation may be present in the gel at a concentration of 0.1 wt-% or greater, or 0.5 wt-% or greater. The divalent cation may be present in the gel at a concentration of 1 wt-% or less. In some embodiments the gel comprises one or more carboxylic acids. The carboxylic acid may comprise a ketone group. The carboxylic acid may have 10 carbon atoms or less. Preferably, the carboxylic acid has 5 carbon atoms. Preferably, the carboxylic acid is levulinic acid.

One example of a suitable aerosol-generating substrate is a gel comprising nicotine and one or more gelling agents. The gel may comprise from 1 wt-% to 4 wt-% nicotine. The gel may comprise from 1 wt-% to 7 wt-% gelling agent. The gel may comprise from 50 wt-% to 70 wt-% glycerol. The gel may comprise a flavorant, such as a tobacco-based extract. The gel composition may comprise a gelling agent forming a solid medium, glycerol dispersed in the solid medium, and nicotine dispersed in the glycerol.

The housing defines a cartridge body having a housing exterior surface and an interior surface defining a housing interior. The housing may further define at least one air inlet and at least one air outlet. An airflow path extends between the least one air inlet and at least one air outlet. The revolver element is disposed along the airflow path to entrain aerosol formed by the revolver element in the heating position. When aerosol is inhaled by a consumer of the aerosol-forming device, air enters the housing through the at least one air inlet, pass though the housing interior and along the airflow path, and leave the housing through the at least one air outlet. The air outlet may form at least a portion of the mouthpiece. The housing or cartridge body may include a mouthpiece that is formed by the at least one air outlet. The housing or cartridge body may include a mouthpiece that is in fluid connection with the at least one air outlet.

An aerosol-forming substrate reservoir is contained within the housing or cartridge body. The housing interior surface may surround the aerosol-forming substrate reservoir. The aerosol-forming substrate reservoir contains a volume of aerosol-forming substrate. The aerosol-forming substrate reservoir contains a volume of aerosol-forming substrate sufficient to form 200 to 300 inhalations or puffs by a consumer. The aerosol-forming substrate reservoir has an outlet aperture.

A bias element applies force or pressure to the volume of aerosol-forming substrate. Force or pressure is sufficient to flow the volume of aerosol-forming substrate towards and through the outlet aperture. Force or pressure is sufficient to flow the volume of solid gel towards and through the outlet aperture. The bias element force may be provided by a spring element pushing against a surface of the volume of aerosol-forming substrate. The bias element force may be provided by a motor which pushes against a surface of the volume of aerosol-forming substrate directly or indirectly. The bias element force may be provided by mechanically pushing against a surface of the volume of aerosol-forming substrate directly or indirectly.

The revolver element is rotatably fixed to the housing interior. The revolver element has two or more chambers disposed about the revolver element. The revolver element may define a flat disc shape with an axis of rotation at the center of the flat disc. The two or more chambers of the revolver element are sequentially aligned with the outlet aperture as the revolver element rotates. Each of the chambers sequentially receive a defined volume of aerosol-forming substrate as the aerosol-forming system operates. The revolver element may rotate in a clockwise manner or a counter clockwise manner.

At least one chamber aligns with the outlet aperture and is filled with aerosol-forming substrate by the bias element applies force or pressure to the volume of aerosol-forming substrate in the aerosol-forming substrate reservoir, and at least one chamber, containing aerosol-forming substrate, is aligned in a heating position. In the heating position, the chamber containing aerosol-forming substrate is aligned with a heating element that may extend through the housing or may be fixed to the aerosol-forming device when mated with the aerosol-forming device. The chambers may extend through the thickness of the revolver element. The chambers may extend through the entire thickness of the revolver element forming an open aperture through the entire thickness of the revolver element. The chambers may extend through only a portion of the thickness of the revolver element forming a cavity with a bottom wall and side walls defining only a portion of the thickness of the revolver element.

The revolver element may include three chambers disposed about the revolver element. The three chambers may be equally spaced apart from each other around the perimeter of the revolver element. The three chambers of the revolver element are sequentially aligned with the outlet aperture as the revolver element rotates. Each of the chambers sequentially receive a defined volume of aerosol-forming substrate as the aerosol-forming system operates.

The revolver element with three chambers includes a cleaning position. The chamber aligned in the cleaning position has just rotated away from the heating position and may have residue remaining in the chamber. In the cleaning position that residue is removed by mechanical or thermal cleaning. The residue may be removed mechanically with a brush element within the housing or cartridge body. The residue may be removed thermally by a second heating element aligned with the chamber with residue in the cleaning position. The chamber with residue may be thermally cleaned by means of pyrolysis to decompose the residue within the chamber. Thermal cleaning may be accomplished by heating the chamber with residue to a temperature in a range from 400 degrees to 1000 degrees Celsius. The housing interior may define a separate cavity surrounding the cleaning position and a separate cleaning air outlet to prevent high temperature volatiles from entering the airflow path to the consumer during consumption.

The revolver element with three chambers includes a deposition position, a heating position, and a cleaning position. The three chambers align with each of the three positions and rotate sequentially through each of the three positions as the revolver element rotates one complete rotation or 360 degrees.

Initially, the first chamber aligns with the outlet aperture to receive aerosol-forming substrate, the second chamber containing aerosol-forming substrate aligns with the heating position, the third chamber containing residue is aligned in the cleaning position. When the aerosol-forming cartridge is mated with the aerosol-forming device, the consumer may activate the heating element and inhale the aerosol with an inhalation puff. Then the consumer may advance the revolver element one increment to move the first chamber containing aerosol-forming substrate to the heating position, and the second chamber with residue into the cleaning position, and the cleaned third chamber into the deposition position. This method is repeated after every inhalation by the consumer. This provides the consumer a uniform metered dose of aerosol with each inhalation.

An aerosol-forming system includes the cartridge, described above, mated with an aerosol-forming device. The aerosol-forming device includes a device housing defining a device interior and a device exterior surface. The device exterior surface comprises a cartridge receiving surface. The device housing includes a heating element fixed to the cartridge receiving surface. The cartridge is replaceably fixed or mated with the cartridge receiving surface. The heating element aligns with the revolver element in a heating position.

The cartridge receiving surface, or the surface of the cartridge contacting the cartridge receiving surface may include a detent element or securing element to hold the cartridge in place on the cartridge receiving surface. The revolver element may include a shaft coupled to a driving element in the aerosol-forming device configured to rotate the revolver element. The shaft is located along the axis of rotation of the revolver element. The cartridge receiving surface may include an aperture for receiving the shaft.

The driving element may include a motor. The driving element may be coupled to the shaft by a gear element. The driving element may be electrically coupled to control electronics. The driving element may be electrically coupled to the power supply.

The heating element may be a resistive heater or an inductive heater. The heating element may be electrically coupled to control electronics. The heating element may be electrically coupled to the power supply.

The heating element is configured to heat the aerosol-forming substrate to at least 200 degrees Celsius within 300 milliseconds. The heating element is configured to heat the aerosol-forming substrate to at least 200 degrees Celsius within 200 milliseconds. The heating element is configured to heat the aerosol-forming substrate to at least 250 degrees Celsius within 200 milliseconds, or within 100 milliseconds, or within 50 milliseconds, or within 25 milliseconds.

The aerosol-generating device may include a controller or control electronics comprising one or more processors (for example, microprocessors). The one or more processors may operate with associated data storage, or memory, for access to processing programs or routines and one or more types of data that may be employed to carry out the illustrative methods. For example, processing programs or routines stored in data storage may include programs or routines for controlling the one or more of the heating elements, driving element, and optional cleaning second heating element, individually controlling one or more of the driving or heating elements, implementing programs or schemes using one or more of the driving or heating elements, and the like.

The computer program products used to implement the processes described herein may be provided using any programmable language, for example, a high-level procedural or object orientated programming language that is suitable for communicating with a computer system. Any such program products may, for example, be stored on any suitable device, for example, a storage media, readable by a general or special purpose program, controller apparatus for configuring and operating the computer when the suitable device is read for performing the procedures described herein. In other words, at least in one embodiment, the aerosol-generating device may be implemented using a non-transitory computer readable storage medium, configured with a computer program, where the storage medium so configured causes the computer to operate in a specific and predefined manner to perform functions described herein.

The exact configuration of the controller of the aerosol-generating device is not limiting and essentially any device capable of providing suitable computing capabilities and control capabilities to implement the method may be used. In view of the above, it will be readily apparent that the functionality may be implemented in any manner as would be known to one skilled in the art. As such, the computer language, the controller, or any other software/hardware which is to be used to implement the processes described herein shall not be limiting on the scope of the systems, processes, or programs (for example, the functionality provided by such processes or programs) described herein. The methods and processes described in this disclosure, including those attributed to the systems, or various constituent components, may be implemented, at least in part, in hardware, software, firmware, or any combination thereof. For example, various embodiments of the techniques may be implemented within one or more processors, including one or more microprocessors, DSPs, ASICs, FPGAs, CPLDs, microcontrollers, or any other equivalent integrated or discrete logic circuitry, as well as any combinations of such components. When implemented in software, the functionality ascribed to the systems, devices, and methods described in this disclosure may be embodied as instructions on a computer-readable medium such as RAM, ROM, NVRAM, EEPROM, FLASH memory, magnetic data storage media, optical data storage media, or the like. The instructions may be executed by one or more processors to support one or more embodiments of the functionality.

The aerosol-forming device may include a second heating element positioned on the cartridge receiving surface to align with a cleaning position of the revolver element. The second heating element is configured to heat to a temperature of 400 degrees Celsius to clean the residue from the chamber aligned with the second heating element. The second heating element is configured to heat to a temperature in a range from 400 degrees to 600 degrees to clean the residue from the chamber aligned with the second heating element. The second heating element is configured to heat to a temperature in a range from 400 degrees to 1000 degrees Celsius to clean the residue from the chamber aligned with the second heating element.

The second heating element may be a resistive heater or an inductive heater. The second heating element may be electrically coupled to control electronics. The second heating element may be electrically coupled to the power supply.

A method of forming an aerosol includes inserting a cartridge, described above, onto the cartridge receiving surface of the aerosol-forming device, described above. Heating the aerosol-forming substrate contained within a chamber of the revolver element of the cartridge to form an aerosol in an inhalation airflow flowing through the cartridge and flowing the aerosol and inhalation airflow through a mouthpiece.

The method may further include rotating the revolver element one increment to align a chamber containing the aerosol-forming substrate or gel over the heating element in the heating position and aligning an empty chamber with the outlet aperture of the aerosol-forming substrate reservoir to deposit aerosol-forming substrate or gel into the empty chamber.

The method may further include rotating the revolver element one increment to align a chamber containing the aerosol-forming substrate or gel over the heating element in the heating position, aligning a chamber containing residue over a cleaning position and mechanically or thermally cleaning the chamber containing residue, and aligning an empty chamber with the outlet aperture of the aerosol-forming substrate reservoir to deposit gel into the empty chamber.

The method may further include replacing the cartridge with a new cartridge once the cartridge is depleted of gel. The cartridge may provide a consumer with 200 to 300 inhalations or puffs.

The invention is defined in the claims. However, below there is provided a non-exhaustive listing of non-limiting examples. Any one or more of the features of these examples may be combined with any one or more features of another example, embodiment, or aspect described herein.

The Examples will now be further described with reference to the figures in which:
**FIG. 1** is a schematic cross-sectional diagram of an illustrative aerosol-forming cartridge;
**FIG. 2** is a transparent schematic top view of a portion of the aerosol-forming cartridge of **FIG.1****;**
**FIG. 3** is a transparent schematic perspective view of the aerosol-forming cartridge of **FIG.1****;**
**FIG. 4** is a schematic cross-sectional diagram of an illustrative aerosol-forming device;
**FIG. 5** is a schematic top view of the aerosol-forming device of **FIG. 4****;**
**FIG. 6** is a schematic cross-sectional diagram of an illustrative aerosol-forming system including the aerosol-forming cartridge of **FIG.1** mated to the aerosol-forming device of **FIG. 4****;** and
**FIG. 7** is schematic side elevation view of the aerosol-forming system of **FIG. 6****.**

The schematic drawings are not necessarily to scale and are presented for purposes of illustration and not limitation. The drawings depict one or more aspects described in this disclosure. However, it will be understood that other aspects not depicted in the drawing fall within the scope and spirit of this disclosure.

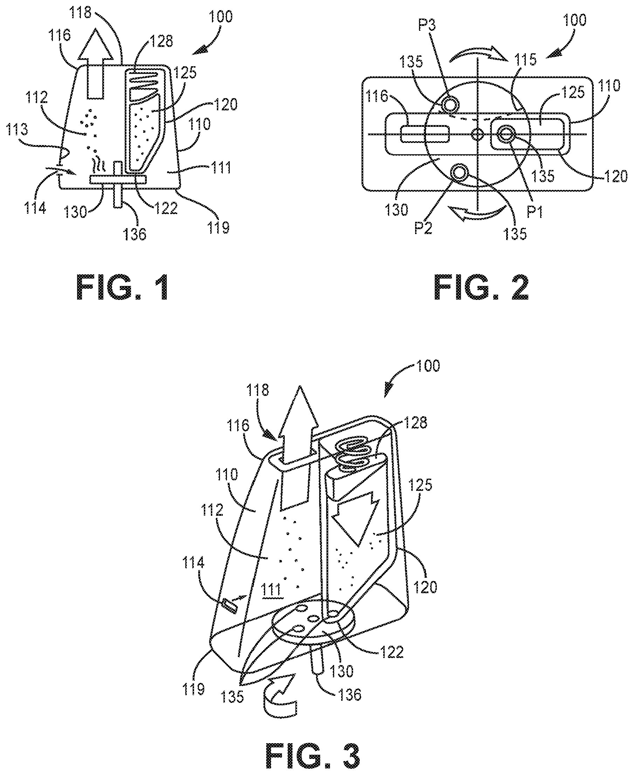

**FIG. 1** is a schematic cross-sectional diagram of an illustrative aerosol-forming cartridge **100.** **FIG. 2** is a transparent schematic top view of a portion of the aerosol-forming cartridge **100** of **FIG.1. FIG. 3** is a transparent schematic perspective view of the aerosol-forming cartridge **100** of **FIG.1****.**

An aerosol-forming cartridge **100** includes a housing **110** defining a cartridge body and having a housing interior **111** defined by an inner surface **113** of the housing **110.** An aerosol-forming substrate reservoir **120** is fixed within the housing interior **111** to the inner surface **113.** A revolver element **130** is rotatably fixed to the housing interior **111** to the inner surface **113.** The revolver element **130** has three chambers **135** disposed about the revolver element **130.** The aerosol-forming substrate reservoir **120** contains a volume of aerosol-forming substrate **125.** The aerosol-forming substrate **125** is a gel. A bias element **128** applies force to the volume of aerosol-forming substrate **125.** The aerosol-forming substrate reservoir **120** has an outlet aperture **122.** The three chambers **135** of the revolver are sequentially aligned with the outlet aperture **122** as the rotates.

The revolver element **130** is a flat disc having a shaft **136** connected to the revolver element 130 at the axis of rotation of the revolver element **130.** The bias element **128** is shown as a spring member that applies a force sufficient to cause the aerosol-forming substrate **125** to flow towards and out of the outlet aperture **122,** depositing aerosol-forming substrate **125** into a chamber **135** aligned with the outlet aperture **122** at a depositing position **P1.** A second chamber **135** is located at the heating position **P2** and a third chamber **135** is located at a cleaning position **P3.** The revolver element **130** rotates clockwise through the three positions **P1, P2** and **P3** when viewing the revolver element **130** from the top view.

The housing **110** includes an airflow path **112** extending from an air inlet **114** through the housing interior **111** to an air outlet **116** the housing. The revolver element **130** is disposed along the airflow path **112,** to provide aerosol into the inhalation air flowing along the airflow path **112.**

The aerosol-forming cartridge **100** includes an aerosol-forming device mating surface **119.** The shaft **136** of the revolver element **130** extends through the aerosol-forming device mating surface **119.** The revolver element **130** may be parallel with the aerosol-forming device mating surface **119.** The aerosol-forming device mating surface **119** may include a heat conduction element configures to transfer heat from the device heating element to the chamber **135** located at the heating position **P2.**

The aerosol-forming cartridge **100** includes a mouthpiece **118.** The air outlet **116** forms a portion of the mouthpiece **118.**

The aerosol-forming cartridge **100** may include a separating wall **115** separating the cleaning position **P3** cavity from the airflow path **112** as shown in **FIG. 2****.** Thermal cleaning may be accomplished by heating the chamber **135** in the cleaning position **P3** to a temperature in a range from 400 degrees to 1000 degrees Celsius. The separating wall **115** prevents high temperature volatiles that are cleaned from the chamber **135** from entering the airflow path **112** to the consumer during consumption.

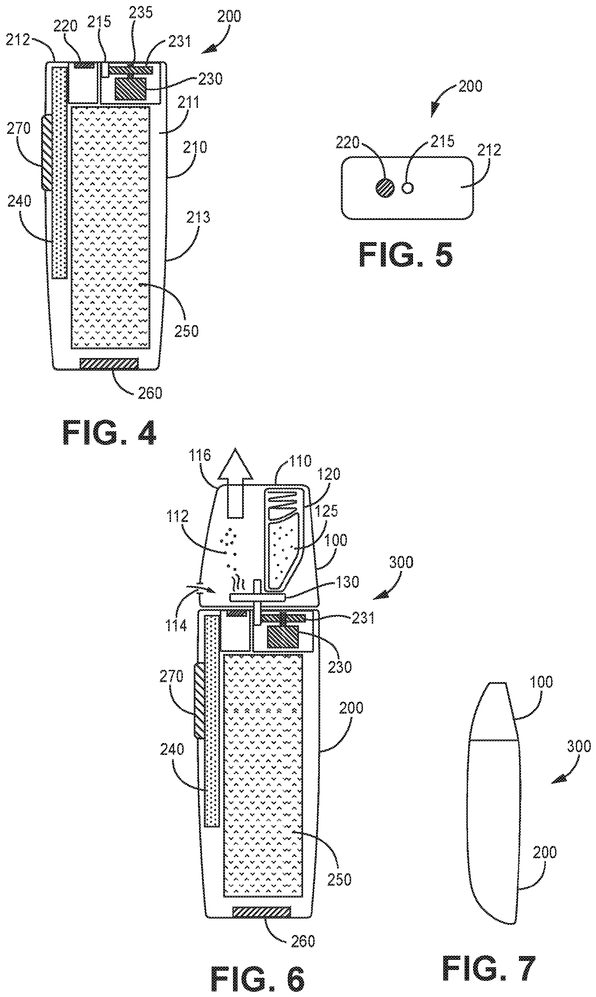

**FIG. 4** is a schematic cross-sectional diagram of an illustrative aerosol-forming device **200.** **FIG. 5** is a schematic top view of the aerosol-forming device **200 of** **FIG. 4. FIG. 6** is a schematic cross-sectional diagram of an illustrative aerosol-forming system **300** including the aerosol-forming cartridge **100 of** **FIG.1** mated to the aerosol-forming device **200 of** **FIG. 4. FIG. 7** is schematic side elevation view of the aerosol-forming system **300 of** **FIG. 6****.**

The aerosol-forming device **200** includes a device housing **210** defining a device interior **211** and a device exterior surface **213,** the device exterior surface **213** including a cartridge receiving surface **212.** A heating element **220** is fixed to the cartridge receiving surface. A cartridge **100** mates with the cartridge receiving surface **212.** The heating element **220** aligns with the revolver element **130** in a gel heating position **P2.**

The cartridge receiving surface **212** includes a shaft aperture **215** that is configured to receive the shaft **136** of the revolver element **130** once the cartridge **100** mates with the cartridge receiving surface **212.** The shaft **136** is coupled to the driving element **230** such as a motor. The motor **230** includes a motor shaft **235** that is coupled to one or more gear elements **231** (illustrated here as a single gear for convenience). The driving element **230** is illustrated coupled to the shaft **136** by the gear elements **231.** The driving element **230** rotates the revolver element **130** to provide aerosol-forming substrate **125** to the heating element **220.**

Control electronics **240** and a power supply **250** are disposed within the device housing **210.** The driving element **230** is electrically coupled to the control electronics **240.** The driving element is electrically coupled to the power supply **250.**

The aerosol-forming device **200** is illustrated including a charging port **260** electrically coupled to the power supply **250** to re-charge the power supply **250.** The aerosol-forming device **200** is illustrated including a power switch and a display unit **270.** The power switch and a display unit **270** is electrically coupled to the control electronics **240.**

For the purpose of the present description and of the appended claims, except where otherwise indicated, all numbers expressing amounts, quantities, percentages, and so forth, are to be understood as being modified in all instances by the term "about." Also, all ranges include the maximum and minimum points disclosed and include any intermediate ranges therein, which may or may not be specifically enumerated herein. In this context, therefore, a number A is understood as A ±2% of A. Within this context, a number A may be considered to include numerical values that are within general standard error for the measurement of the property that the number A modifies. Also, all ranges include the maximum and minimum points disclosed and include any intermediate ranges therein, which may or may not be specifically enumerated herein.

## Claims

1. An aerosol-forming cartridge (100) comprising:
a housing (110) defining a cartridge body and having a housing interior (111);
an aerosol-forming substrate reservoir (120) fixed within the housing interior (111), the aerosol-forming substrate reservoir (120) containing a volume of aerosol-forming substrate (125), the aerosol-forming substrate being a gel, and a bias element (128) applying force to the volume of aerosol-forming substrate (125), the aerosol-forming substrate reservoir (120) having an outlet aperture (122); and
a revolver element (130) rotatably fixed to the housing interior (111) and having two or more chambers (135) disposed about the revolver element (130), the two or more chambers (135) are sequentially aligned with the outlet aperture (122) as the revolver element (130) rotates.

2. The aerosol-forming cartridge (100) according to claim 1, wherein the gel comprises nicotine or a nicotine salt.

3. The aerosol-forming cartridge (100) according to any preceding claim, wherein the gel comprises one or more gelling agents comprising a biopolymer, preferably glycerol.

4. The aerosol-forming cartridge (100) according to any preceding claim, wherein the gel comprises 50 wt% to 95 wt% glycerol.

5. The aerosol-forming cartridge (100) according to any preceding claim, wherein the revolver element (130) comprises three chambers (135) that extent through a thickness of the revolver element (130).

6. The aerosol-forming cartridge (100) according to any preceding claim, wherein the housing (110) comprises an airflow path (112) extending from an air inlet (114) through the housing interior (111) to an air outlet (116) of the housing (110), and the revolver element (130) is disposed along the airflow path (112), and the air outlet (116) forms at least a portion of a mouthpiece (118).

7. The aerosol-forming cartridge (100) according to any preceding claim, wherein the revolver element (130) comprises three chambers (135), wherein a first chamber (135) aligns with the outlet aperture (122), a second chamber (135) aligns in a heating position (P2), and a third chamber (135) aligns with a cleaning position (P3),
wherein the third chamber (135) aligns with a cleaning position in which it is configured to be mechanically cleaned or thermally cleaned.

8. An aerosol-forming system (300) comprising:
an aerosol-forming device (200) comprising:
a device housing (210) defining a device interior (211) and a device exterior surface (213), the device exterior surface (213) comprises a cartridge receiving surface (212);
a heating element (220) fixed to the cartridge receiving surface (212); and
a cartridge (100) according to any preceding claim configured to mate with the cartridge receiving surface (212);
wherein the heating element (220) aligns with the revolver element (130) in a gel heating position (P2).

9. The aerosol-forming system (300) according to claim 8, wherein the revolver element (130) comprises a shaft (136) coupled to a driving element (230) in the aerosol-forming device (200) configured to rotate the revolver element (130).

10. The aerosol-forming system (300) according to claim 9, wherein the driving element (230) and the heating element (220) are electrically coupled to control electronics (240) within the aerosol-forming device (200), and the driving element (230) and the heating element (220) are electrically coupled to a power supply (250) within the aerosol-forming device (200).

11. The aerosol-forming system (300) according to one of claims 8 to 10, wherein the heating element (220) is a resistive heating element configured to heat the aerosol-forming substrate to 200 degrees C within 300 milliseconds.

12. The aerosol-forming system (300) according to one of claims 8 to 11, wherein the aerosol-forming device (200) comprises a second heating element configured to align with a cleaning position (P3) of the revolver element (130).

13. A method of forming an aerosol, comprising:
inserting a cartridge (100) of any one of claim 1 to 9 onto the cartridge receiving surface (212) of the aerosol-forming device (200) of any one of claims 10 to 14;
heating the aerosol-forming substrate (125) contained within a chamber (135) of the revolver element (130) to form an aerosol in an inhalation airflow flowing through the cartridge (100); and
flowing the aerosol and inhalation airflow through a mouthpiece (118).

14. The method according to claim 13, further comprising rotating the revolver element (130) one increment to align a chamber (135) containing the aerosol-forming substrate (125) over the heating element (220) in the heating position and aligning an empty chamber (135) with the aerosol-forming substrate outlet aperture (122) of the aerosol-forming substrate reservoir to deposit aerosol-forming substrate (125) into the empty chamber (135).

15. The method according to claim 13, further comprising rotating the revolver element (130) one increment to align a chamber (135) with residue with a brush or second heater to clean the chamber (135) after forming the aerosol in an inhalation airflow flowing through the cartridge (100) and forming a cleaned chamber (135).

## Patentansprüche

1. Aerosolbildende Patrone (100) aufweisend:
ein Gehäuse (110), das einen Patronenkörper definiert und ein Gehäuseinneres (111) aufweist;
einen Vorratsbehälter für das aerosolbildende Substrat (120), der innerhalb des Gehäuseinneren (111) befestigt ist, wobei der Vorratsbehälter für das aerosolbildende Substrat (120) ein Volumen des aerosolbildenden Substrats (125) enthält, das aerosolbildende Substrat ein Gel ist, und ein Vorspannelement (128) eine Kraft auf das Volumen des aerosolbildenden Substrats (125) aufbringt, wobei der Vorratsbehälter für das aerosolbildende Substrat (120) eine Auslassöffnung (122) aufweist; und
ein Revolverelement (130), das drehbar an dem Gehäuseinneren (111) befestigt ist und zwei oder mehr Kammern (135) aufweist, die um das Revolverelement (130) herum angeordnet sind, wobei die zwei oder mehr Kammern (135) nacheinander mit der Auslassöffnung (122) ausgerichtet sind, wenn sich das Revolverelement (130) dreht.

2. Aerosolbildende Patrone (100) nach Anspruch 1, wobei das Gel Nikotin oder ein Nikotinsalz umfasst.

3. Aerosolbildende Patrone (100) nach einem beliebigen vorhergehenden Anspruch, wobei das Gel ein oder mehrere Geliermittel umfasst, die ein Biopolymer, bevorzugt Glycerin, umfassen.

4. Aerosolbildende Patrone (100) nach einem beliebigen vorhergehenden Anspruch, wobei das Gel 50 Gew.-% bis 95 Gew.-% Glycerin umfasst.

5. Aerosolbildende Patrone (100) nach einem beliebigen vorhergehenden Anspruch, wobei das Revolverelement (130) drei Kammern (135) aufweist, die sich über eine Dicke des Revolverelements (130) erstrecken.

6. Aerosolbildende Patrone (100) nach einem beliebigen vorhergehenden Anspruch, wobei das Gehäuse (110) einen Luftstrompfad (112) aufweist, der sich von einem Lufteinlass (114) durch das Gehäuseinnere (111) zu einem Luftauslass (116) des Gehäuses (110) erstreckt, und das Revolverelement (130) entlang des Luftstrompfades (112) angeordnet ist, und der Luftauslass (116) wenigstens einen Abschnitt eines Mundstücks (118) bildet.

7. Aerosolbildende Patrone (100) nach einem beliebigen vorhergehenden Anspruch, wobei das Revolverelement (130) drei Kammern (135) aufweist, wobei sich eine erste Kammer (135) auf die Auslassöffnung (122) ausrichtet, sich eine zweite Kammer (135) auf eine Erwärmungsposition (P2) ausrichtet und sich eine dritte Kammer (135) auf eine Reinigungsposition (P3) ausrichtet,
wobei sich die dritte Kammer (135) auf eine Position ausrichtet, in der sie für ein mechanisches Reinigen oder thermisches Reinigen eingerichtet ist.

8. Aerosolbildendes System (300) aufweisend:
eine aerosolbildende Vorrichtung (200) aufweisend:
ein Vorrichtungsgehäuse (210), das ein Vorrichtungsinneres (211) und eine Vorrichtungsaußenfläche (213) definiert, wobei die Vorrichtungsaußenfläche (213) eine Patronenaufnahmefläche (212) aufweist;
ein Heizelement (220), das an der Patronenaufnahmefläche (212) befestigt ist; und
eine Patrone (100) nach einem beliebigen vorhergehenden Anspruch, die für ein Verbinden mit der Patronenaufnahmefläche (212) eingerichtet ist;
wobei das Heizelement (220) mit dem Revolverelement (130) in einer Gelerwärmungsposition (P2) ausgerichtet ist.

9. Aerosolbildendes System (300) nach Anspruch 8, wobei das Revolverelement (130) eine Welle (136) aufweist, die mit einem Antriebselement (230) in der aerosolbildenden Vorrichtung (200) gekoppelt ist, die für ein Drehen des Revolverelements (130) eingerichtet ist.

10. Aerosolbildendes System (300) nach Anspruch 9, wobei das Antriebselement (230) und das Heizelement (220) elektrisch mit einer Steuerelektronik (240) innerhalb der aerosolbildenden Vorrichtung (200) gekoppelt sind und das Antriebselement (230) und das Heizelement (220) elektrisch mit einer Energieversorgung (250) innerhalb der aerosolbildenden Vorrichtung (200) gekoppelt sind.

11. Aerosolbildendes System (300) nach einem der Ansprüche 8 bis 10, wobei das Heizelement (220) ein Widerstandsheizelement ist, das dazu eingerichtet ist, das aerosolbildende Substrat innerhalb von 300 Millisekunden auf 200 Grad C zu erwärmen.

12. Aerosolbildendes System (300) nach einem der Ansprüche 8 bis 11, wobei die aerosolbildende Vorrichtung (200) ein zweites Heizelement aufweist, das dazu eingerichtet ist, sich mit einer Reinigungsposition (P3) des Revolverelements (130) auszurichten.

13. Verfahren für ein Bilden eines Aerosols, umfassend:
Einsetzen einer Patrone (100) nach einem der Ansprüche 1 bis 9 auf die Patronenaufnahmefläche (212) der aerosolbildenden Vorrichtung (200) nach einem der Ansprüche 10 bis 14;
Erwärmen des aerosolbildenden Substrats (125), das innerhalb einer Kammer (135) des Revolverelements (130) enthalten ist, um ein Aerosol in einem durch die Patrone (100) strömenden Inhalationsluftstrom zu bilden; und
Strömen des Aerosols und des Inhalationsluftstroms durch ein Mundstück (118).

14. Verfahren nach Anspruch 13, ferner umfassend Drehen des Revolverelements (130) um ein Inkrement, um eine Kammer (135), die das aerosolbildende Substrat (125) enthält, über dem Heizelement (220) in der Heizposition auszurichten, und Ausrichten einer leeren Kammer (135) mit der Auslassöffnung (122) des Vorratsbehälters für aerosolbildendes Substrat, um das aerosolbildende Substrat (125) in die leere Kammer (135) abzugeben.

15. Verfahren nach Anspruch 13, ferner umfassend Drehen des Revolverelements (130) um ein Inkrement, um eine Kammer (135) mit Rückständen mit einer Bürste oder einer zweiten Heizvorrichtung für ein Reinigen der Kammer (135) nach der Bildung des Aerosols in einem durch die Patrone (100) strömenden Inhalationsluftstrom zu reinigen und eine gereinigte Kammer (135) zu bilden.

## Revendications

1. Cartouche formant aérosol (100) comprenant :
un logement (110) définissant un corps de cartouche et ayant un intérieur de logement (111) ;
un réservoir de substrat formant aérosol (120) fixé au sein de l'intérieur de logement (111), le réservoir de substrat formant aérosol (120) contenant un volume de substrat formant aérosol (125), le substrat formant aérosol étant un gel, et un élément de sollicitation (128) appliquant une force au volume de substrat formant aérosol (125), le réservoir de substrat formant aérosol (120) ayant une ouverture de sortie (122) ; et
un élément barillet (130) fixé de manière rotative à l'intérieur de logement (111) et ayant deux chambres (135) ou plus disposées autour de l'élément barillet (130), les deux chambres (135) ou plus sont alignées séquentiellement avec l'ouverture de sortie (122) à mesure que l'élément barillet (130) tourne.

2. Cartouche formant aérosol (100) selon la revendication 1, dans laquelle le gel comprend de la nicotine ou un sel de nicotine.

3. Cartouche formant aérosol (100) selon l'une quelconque des revendications précédentes, dans laquelle le gel comprend un ou plusieurs agents gélifiants comprenant un biopolymère, de préférence du glycérol.

4. Cartouche formant aérosol (100) selon l'une quelconque des revendications précédentes, dans laquelle le gel comprend 50 % en poids à 95 % en poids de glycérol.

5. Cartouche formant aérosol (100) selon l'une quelconque des revendications précédentes, dans laquelle l'élément barillet (130) comprend trois chambres (135) qui s'étendent à travers une épaisseur de l'élément barillet (130).

6. Cartouche formant aérosol (100) selon l'une quelconque des revendications précédentes, dans laquelle le logement (110) comprend un trajet d'écoulement d'air (112) s'étendant depuis une entrée d'air (114) à travers l'intérieur de logement (111) jusqu'à une sortie d'air (116) du logement (110), et l'élément barillet (130) est disposé le long du trajet d'écoulement d'air (112), et la sortie d'air (116) forme au moins une portion d'un embout buccal (118).

7. Cartouche formant aérosol (100) selon l'une quelconque des revendications précédentes, dans laquelle l'élément barillet (130) comprend trois chambres (135), dans laquelle une première chambre (135) s'aligne avec l'ouverture de sortie (122), une deuxième chambre (135) s'aligne dans une position de chauffage (P2), et une troisième chambre (135) s'aligne avec une position de nettoyage (P3),
dans laquelle la troisième chambre (135) s'aligne avec une position de nettoyage dans laquelle elle est configurée pour être nettoyée mécaniquement ou nettoyée thermiquement.

8. Système formant aérosol (300) comprenant :
un dispositif formant aérosol (200) comprenant :
un logement de dispositif (210) définissant un intérieur
de dispositif (211) et une surface extérieure de dispositif (213), la surface extérieure de dispositif (213) comprend une surface de réception de cartouche (212) ;
un élément de chauffage (220) fixé à la surface de réception de cartouche (212) ; et
une cartouche (100) selon l'une quelconque des revendications précédentes configurée pour s'accoupler à la surface de réception de cartouche (212) ;
dans lequel l'élément de chauffage (220) s'aligne avec l'élément barillet (130) dans une position de chauffage de gel (P2).

9. Système formant aérosol (300) selon la revendication 8, dans lequel l'élément barillet (130) comprend un arbre (136) couplé à un élément d'entraînement (230) dans le dispositif formant aérosol (200) configuré pour faire tourner l'élément barillet (130).

10. Système formant aérosol (300) selon la revendication 9, dans lequel l'élément d'entraînement (230) et l'élément de chauffage (220) sont couplés électriquement pour commander l'électronique (240) au sein du dispositif formant aérosol (200), et l'élément d'entraînement (230) et l'élément de chauffage (220) sont couplés électriquement à une alimentation électrique (250) au sein du dispositif formant aérosol (200).

11. Système formant aérosol (300) selon l'une des revendications 8 à 10, dans lequel l'élément de chauffage (220) est un élément de chauffage résistif configuré pour chauffer le substrat formant aérosol à 200 degrés C en 300 millisecondes.

12. Système formant aérosol (300) selon l'une des revendications 8 à 11, dans lequel le dispositif formant aérosol (200) comprend un deuxième élément de chauffage configuré pour s'aligner avec une position de nettoyage (P3) de l'élément barillet (130).

13. Procédé de formation d'un aérosol comprenant :
l'insertion d'une cartouche (100) selon l'une quelconque des revendications 1 à 9 sur la surface de réception de cartouche (212) du dispositif formant aérosol (200) selon l'une quelconque des revendications 10 à 14 ;
le chauffage du substrat formant aérosol (125) contenu au sein d'une chambre (135) de l'élément barillet (130) pour former un aérosol dans un écoulement d'air d'inhalation s'écoulant à travers la cartouche (100) ; et
l'écoulement de l'aérosol et de l'écoulement d'air d'inhalation à travers un embout buccal (118).

14. Procédé selon la revendication 13, comprenant en outre la rotation de l'élément barillet (130) d'un incrément pour aligner une chambre (135) contenant le substrat formant aérosol (125) sur l'élément de chauffage (220) dans la position de chauffage et l'alignement d'une chambre vide (135) avec l'ouverture de sortie de substrat formant aérosol (122) du réservoir de substrat formant aérosol pour déposer le substrat formant aérosol (125) jusque dans la chambre vide (135).

15. Procédé selon la revendication 13, comprenant en outre la rotation de l'élément barillet (130) d'un incrément pour aligner une chambre (135) avec un résidu avec une brosse ou un deuxième dispositif de chauffage pour nettoyer la chambre (135) après la formation de l'aérosol dans un écoulement d'air d'inhalation s'écoulant à travers la cartouche (100) et formant une chambre nettoyée (135).
